# NEUE EUROPÄISCHE PATENTSCHRIFT

(11) **EP 2 392 247 B2**
(45) Veröffentlichungstag und Bekanntmachung des Hinweises auf die Entscheidung über den Einspruch: **03.07.2019**
(45) Hinweis auf die Patenterteilung: 12.10.2016
(21) Anmeldenummer: 11167768.8
(22) Anmeldetag: 26.05.2011
(51) Int. Cl.: A61B 1/00, G02B 23/24, G02B 7/02

(54) **Medizinisches Instrument mit einem Fenster**
Medicinal instrument with a window
Instrument médical doté d'une fenêtre

(30) Priorität: 01.06.2010 DE 102010022432
(43) Veröffentlichungstag der Anmeldung: 07.12.2011
(73) Patentinhaber: Karl Storz SE & Co. KG, 78532 Tuttlingen (DE)
(72) Erfinder: Baumann, Harald, 78532 Tuttlingen (DE); Teichtmann, Elmar, 78532 Tuttlingen (DE)
(74) Vertreter: Wimmer, Stephan

(56) Entgegenhaltungen:
- EP-A1- 2 105 777
- DE-A1- 19 644 729
- DE-A1- 19 941 320
- DE-C1- 19 836 285
- DE-U1- 9 412 590
- US-A- 4 669 818
- US-A- 4 779 613
- US-A1- 2006 235 274
- US-B1- 6 547 721
- US-B1- 6 767 322

## Beschreibung

Die vorliegende Erfindung bezieht sich auf ein medizinisches Instrument mit einem Fensterbauteil, das eine Öffnung verschließt, und auf ein Verfahren zum Herstellen eines medizinischen Instruments. Das medizinische Instrument ist insbesondere ein Endoskop.

Medizinische Instrumente müssen vor jeder Verwendung gereinigt und sterilisiert werden. Die Sterilisation erfolgt in der Regel durch Autoklavieren, also in einer reinen Wasserdampfatmosphäre bei Überdruck und Temperaturen deutlich über 100° Celsius. Ein Eindringen von Wasserdampf in ein Endoskop oder ein anderes optisches medizinisches Instrument hätte nachteilige Folgen für dessen optische Eigenschaften.

Optische medizinische Instrumente sind deshalb in der Regel hermetisch dicht verschlossen. Öffnungen, durch die beispielsweise am distalen und am proximalen Ende eines Endoskops Licht ein- und austreten muss, sind durch Fensterbauteile aus einem transparenten Material verschlossen. Viele Fensterbauteile bestehen aus Saphir, der sich durch große Härte und chemische Resistenz auszeichnet. Zur Bildung einer hermetisch dichten Verbindung kann der Rand eines Saphir-Fensters mittels Lot mit dem Gehäuse gefügt werden. Saphir ist allerdings (einachsig) doppelbrechend und deshalb beispielsweise für diagnostische Verfahren, die polarisiertes Licht verwenden, nicht uneingeschränkt geeignet.

Gläser sind nicht doppelbrechend, werden jedoch häufig schon beim Abkühlen nach dem Lötvorgang durch thermisch hervorgerufene mechanische Spannungen zerstört. Ein weiteres Problem besteht darin, dass einige herkömmliche Glaslote mit vorteilhaften mechanischen und auf die Fertigung bezogenen Eigenschaften nicht biokompatibel sind. Diese Glaslote können deshalb nicht ohne Weiteres für medizinische Instrumente verwendet werden, die mit dem menschlichen Körper in Kontakt kommen.

Eine Vorrichtung gemäß dem Oberbegriff des unabhängigen Anspruchs 1 ist aus der Druckschrift DE 198 36 285 C1 bekannt.

Eine Aufgabe der vorliegenden Erfindung besteht darin. ein verbessertes medizinisches Instrument mit einem Fensterbauteil und ein verbessertes Verfahren zum Herstellen eines medizinischen Instruments mit einem Fensterbauteil zu schaffen.

Diese Aufgabe wird durch die Gegenstände der unabhängigen Ansprüche gelöst.

Weiterbildungen sind in den abhängigen Ansprüchen angegeben.

Ausführungsformen der vorliegenden Erfindung beruhen auf der Idee, am Rand einer Öffnung eines medizinischen Instruments eine Lippe vorzusehen, deren innerer Rand am Rand des Fensterbauteils anliegt. Diese Lippe trennt dann beispielsweise ein Lot an ihrer Innenseite, das eine hermetische Dichtheit gewährleistet, von einem Klebstoff an ihrer Außenseite, das die Biokompatibilität sicherstellt.

Die vorliegende Erfindung beruht insbesondere auf der Idee. an einem medizinischen Instrument mit einem Fensterbauteil einen das Fensterbauteil umschließenden Bereich als Festkörpergelenk auszubilden. In dem Festkörpergelenk können mechanische Spannungen abgebaut werden, die somit das Fensterbauteil nicht mehr gefährden können. Für das Fensterbauteil können deshalb Materialien verwendet werden, die herkömmlich durch thermisch hervorgerufene mechanische Spannungen zerstört würden und deshalb nicht verwendet werden können.

Ein medizinisches Instrument umfasst ein Gehäuse mit einer Öffnung, ein Fensterbauteil, das ein transparentes Material aufweist und die Öffnung verschließt, und einen dünnwandigen Bereich, der die Öffnung umgibt.

Der dünnwandige Bereich des Gehäuses ist dünnwandiger und insbesondere wesentlich dünnwandiger als andere Bereiche des Gehäuses. Die Wandstärke des Gehäuses beträgt im dünnwandigen Bereich beispielsweise nur ein Drittel, ein Fünftel oder ein Zehntel der mittleren Wandstärke des Gehäuses.

Der dünnwandige Bereich kann auf mehrere nachfolgend beschriebene Weisen die mechanische Belastung des Fensterbauteils reduzieren und/oder die Herstellung des medizinischen Instruments vereinfachen.

Bei einem medizinischen Instrument, wie es hier beschrieben ist, kann der dünnwandige Bereich eine Lippe mit einem Rand, der an einer Randfläche des Fensterbauteils anliegt, umfassen.

Insbesondere mit den nachfolgend beschriebenen Merkmalen kann das Fensterbauteil die Öffnung in dem Gehäuse hermetisch dicht verschließen. Die Lippe liegt insbesondere in einer Ebene oder im Wesentlichen in einer Ebene, deren Normale parallel oder im Wesentlichen parallel zur Längsachse des medizinischen Instruments ist. Die Lippe ist dünner bzw. weist eine geringere Randstärke auf als andere Bereiche des Gehäuses. Insbesondere weist die Lippe eine Dicke von weniger als 0,5 mm oder von weniger als 0,3 mm auf. Die Lippe ist insbesondere einstückig mit dem Gehäuse ausgebildet. Die Lippe erstreckt sich insbesondere entlang dem gesamten Rand der Öffnung. Alle lokalen Flächennormalen der Randfläche liegen insbesondere in einer Ebene, in der die Öffnung liegt. Die Randfläche des Fensterbauteils, an der der Rand der Lippe anliegt, ist insbesondere die Mantelfläche eines Kreiszylinders oder eines anderen Zylinders.

Abhängig von ihrer Ausgestaltung kann die Lippe alternativ oder gleichzeitig mehrere Funktionen erfüllen. Insbesondere bei einer dünnwandigen Ausgestaltung der Lippe kann diese geeignet sein, mechanische Spannungen aufzunehmen und so die mechanischen Spannungen, denen das Fensterbauteil beispielsweise beim Abkühlen nach einem Lötvorgang ausgesetzt ist, reduzieren. Insbesondere wenn der radial innere Rand der Lippe nicht an einem Rand der Randfläche des Fensterbauteils, sondern in einem mittleren Bereich der Randfläche anliegt, kann die Lippe geeignet sein, Lot an ihrer Innenseite von Klebstoff an ihrer Außenseite zu isolieren. Mit der Innenseite der Lippe ist die dem Innenraum des Gehäuses zugewandte Seite der Lippe gemeint. Mit der Außenseite der Lippe ist die der Umgebung des Gehäuses zugewandte Seite der Lippe gemeint.

Wie bereits erwähnt, kann bei einem medizinischen Instrument, wie es hier beschrieben ist, innerhalb der Lippe ein Lot einen Rahmen um die Öffnung und die Randfläche des Fensterbauteils stoffschlüssig verbinden.

Wie ebenfalls bereits erwähnt wurde, kann bei einem medizinischen Instrument, wie es hier beschrieben ist, außerhalb der Lippe ein Klebstoff den Rahmen um die Öffnung und die Randfläche des Fensterbauteils stoffschlüssig verbinden.

Der Rahmen wird insbesondere durch das Gehäuse gebildet bzw. ist ein einstückiger bzw. integraler Bestandteil des Gehäuses. Das Lot ist insbesondere nicht nur mit dem Rahmen und der Randfläche des Fensterbauteils, sondern auch mit der Innenseite der Lippe stoffschlüssig verbunden oder grenzt an diese zumindest an. Der Klebstoff ist insbesondere nicht nur mit dem Rahmen und der Randfläche, sondern auch mit der Außenseite der Lippe stoffschlüssig verbunden oder grenzt an diese zumindest an.

Eine stoffschlüssige Verbindung des Rahmens und der Randfläche des Fensterbauteils mittels eines Lots kann eine hermetische Dichtheit der Fügestelle gewährleisten. Die Lippe kann einen unmittelbaren Kontakt zwischen dem Lot und der Umgebung des medizinischen Instruments vermeiden. Dies kann, wenn das Lot nicht biokompatibel ist, eine Verwendung des medizinischen Instruments im menschlichen Körper ermöglichen.

Eine weitere Verbesserung ist möglich, indem gleichzeitig außerhalb der Lippe der Klebstoff vorgesehen ist. Dieser kann ebenfalls zur räumlichen Trennung zwischen dem Lot und der Umgebung des medizinischen Instruments beitragen. Bei gleichzeitiger Verwendung eines Lots innerhalb der Lippe und eines Klebstoffs außerhalb der Lippe kann die Lippe eine Diffusion von Bestandteilen des Lots in den Klebstoff oder umgekehrt verhindern oder zumindest einschränken. Dadurch können manche Kombinationen von Loten und Klebstoffen, die jeweils für sich vorteilhafte Eigenschaften aufweisen, erst ermöglicht werden, weil eine gegenseitige Zerstörung verhindert oder zumindest deutlich verlangsamt ist.

Bei einem medizinischen Instrument, wie es hier beschrieben ist, umfasst der dünnwandige Bereich des Gehäuses ein Festkörpergelenk.

Der als Festkörpergelenk ausgebildete Bereich des Gehäuses ist insbesondere der Gestalt des Rands des Fensterbauteils oder ggf. des Rahmens ähnlich und/oder weist einen konstanten Abstand vom Rand des Fensterbauteils oder ggf. vom Rahmen auf. Das Festkörpergelenk weist eine im Vergleich zu anderen Bereichen des Gehäuses deutlich geringere Wandstärke und damit eine deutlich höhere Elastizität auf. Insbesondere beträgt die Wandstärke im Bereich des Festkörpergelenks höchstens ein Drittel oder höchstens ein Fünftel oder höchstens ein Zehntel der Wandstärke in einem Bereich, der sich in einer von der Öffnung abgewandten Richtung an das Festkörpergelenk anschließt, auf.

Das Festkörpergelenk kann mechanische Spannungen abbauen bzw. verhindern oder reduzieren, die andernfalls beispielsweise beim Abkühlen des Gehäuses nach dem Einlöten des Fensterbauteils aufgrund unterschiedlicher thermischer Ausdehnungskoeffizienten der Materialien des Gehäuses und des Fensterbauteils zur Zerstörung des Fensterbauteils führen könnten. Dadurch wird das Spektrum der möglichen Kombinationen von Materialien für das Gehäuse und von transparenten Materialien für das Fensterbauteil deutlich erweitert.

Die minimale Wandstärke des Gehäuses liegt insbesondere im Bereich des Festkörpergelenks vor.

Die minimale Wandstärke des Gehäuses beträgt insbesondere höchstens 0,5 mm oder höchstens 0,3 mm. Insbesondere bei Edelstahl als Material des Gehäuses kann eine minimale Wandstärke von 0,2 mm im Bereich des Festkörpergelenks bei ausreichender Flexibilität eine ausreichende Robustheit bieten.

Bei einem medizinischen Instrument, wie es hier beschrieben ist, kann das Fensterbauteil ein amorphes Material aufweisen.

Insbesondere kann das Fensterbauteil ein Glas aufweisen. Vor allem durch das Festkörpergelenk und/oder durch die dünnwandige Lippe kann die mechanische Belastung des Fensterbauteils so weit reduziert werden, dass auch amorphe Materialien mit ihren teilweise deutlich schlechteren mechanischen Eigenschaften für das Fensterbauteil verwendbar sind. Damit kann beispielsweise auf doppelbrechenden Saphir verzichtet werden. Das breite Spektrum optischer Eigenschaften einer Vielfalt von Gläsern kann neue Verwendungen des medizinischen Instruments ermöglichen, beispielsweise neue diagnostische Methoden.

Ein medizinisches Instrument, wie es hier beschrieben ist, ist insbesondere ein Endoskop, wobei das Gehäuse einen tubusförmigen Abschnitt aufweist, an dessen Ende die Öffnung angeordnet ist.

Der tubusförmige Abschnitt ist insbesondere am proximalen Ende des Endoskops angeordnet bzw. bildet das proximale Ende des Endoskops. Das Fensterbauteil ist in diesem Fall beispielsweise zur Auskopplung von Licht aus dem Endoskop ausgebildet und Bestandteil eines Okulars oder zur optischen Kopplung mit einem Okular oder einer Kamera vorgesehen. Die beschriebene Problematik mechanischer Spannungen, die durch unterschiedliche thermische Ausdehnungskoeffizienten hervorgerufen werden, ist oft am proximalen Ende eines Endoskops besonders bedeutend, weil dort die Öffnung und das Fensterbauteil typischerweise deutlich größer sind als am distalen Ende eines Endoskops. Bei einem Verfahren zum Herstellen eines medizinischen Instruments werden ein Gehäuse bereitgestellt, eine Öffnung und ein dünnwandiger Bereich, der die Öffnung umgibt, ausgebildet, und ein Fensterbauteil, das ein transparentes Material aufweist, in die Öffnung eingesetzt.

Bei einem Verfahren, wie es hier beschrieben ist, kann der dünnwandige Bereich so ausgebildet werden, dass er eine Lippe umfasst, wobei das Fensterbauteil derart in die Öffnung eingesetzt wird, dass ein Rand der Lippe an einer Randfläche des Fensterbauteils anliegt.

Bei einem Verfahren, wie es hier beschrieben ist, kann die Randfläche des Fensterbauteils mit dem Gehäuse mittels eines Lots innerhalb der Lippe stoffschlüssig verbunden werden. Bei einem Verfahren, wie es hier beschrieben ist, kann die Randfläche des Fensterbauteils mittels eines Klebstoffs außerhalb der Lippe stoffschlüssig verbunden werden.

Bei einem Verfahren. wie es hier beschrieben ist, umfasst der dünnwandige Bereich ein Festkörpergelenk.

Die hier beschriebenen Verfahren sind insbesondere zum Herstellen eines medizinischen Instruments, wie es hier beschrieben ist, ausgebildet.

### Kurzbeschreibung der Figuren

Nachfolgend werden Ausführungsformen anhand der beigefügten Figuren näher erläutert. Es zeigen:
- Figur 1: eine schematische Darstellung eines Endoskops;
- Figur 2: eine schematische Darstellung einer Ausführungsform des proximalen Endes des Endoskops aus Figur 1;
- Figur 3: eine schematische Darstellung einer weiteren Ausführungsform des proximalen Endes des Endoskops aus Figur 1;
- Figur 4: eine schematische Darstellung einer weiteren Ausführungsform des proximalen Endes des Endoskops aus Figur 1;
- Figur 5: eine weitere schematische Darstellung des proximalen Endes aus Figur 4;
- Figur 6: ein schematisches Flussdiagramm eines Verfahrens zum Herstellen eines medizinischen Instruments.

### Beschreibung der Ausführungsformen

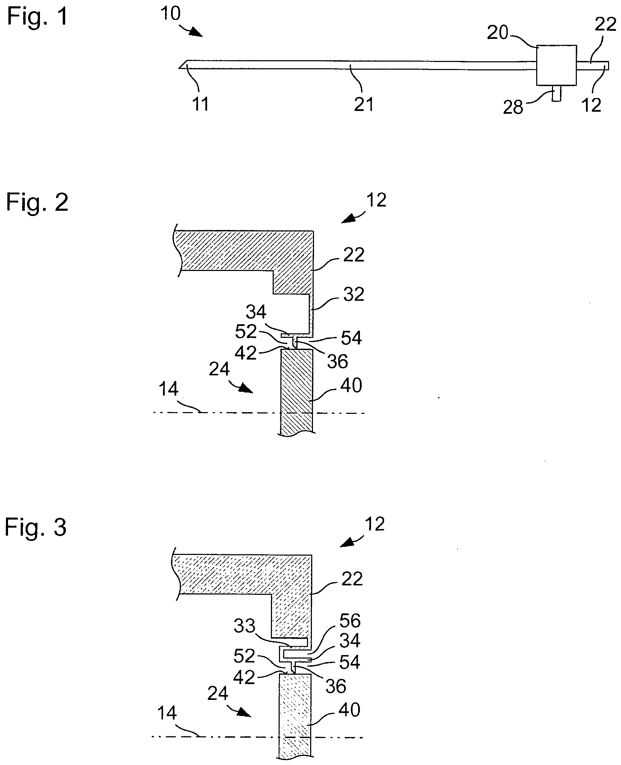

Figur 1 zeigt eine schematische Darstellung eines Endoskops 10 mit einem distalen Ende 11 und einem proximalen Ende 12. Das Endoskop 10 weist ein Gehäuse 20 mit einem langen starren oder flexiblen Schaft 21, der sich bis zum distalen Ende 11 des Endoskops 10 erstreckt, und einem tubusförmigen Abschnitt 22 am proximalen Ende 12 auf. Ferner umfasst das Endoskop 10 einen Anschluss 28 für ein Lichtleitkabel.

Die Figuren 2 bis 5 zeigen schematische Schnittdarstellungen verschiedener Ausführungsformen des distalen Endes 12 des Endoskops 10 aus Figur 1. Die dargestellten Schnittebenen enthalten jeweils die Längsachse 14 des Endoskops oder zumindest des tubusförmigen Abschnitts 22. Im Fall einer Rotationssymmetrie von Teilen des Endoskops 10 (insbesondere des Schafts oder des tubusförmigen Abschnitts 22 des Gehäuses 20) kann die Längsachse 14 gleichzeitig Symmetrieachse sein.

Figur 2 zeigt eine Ausführungsform des proximalen Endes 12 des Endoskops 10, bei der der tubusförmige Abschnitt 22 des Gehäuses 20 ein Festkörpergelenk 32, einen Rahmen 34 und eine Lippe 36 aufweist. Das Festkörpergelenk 32, der Rahmen 34 und die Lippe 36 sind mit dem tubusförmigen Abschnitt 22 des Gehäuses 20 einstückig ausgeführt. Das Festkörpergelenk 32, der Rahmen 34 und die Lippe 36 umgeben die Öffnung 24 jeweils ringförmig. Das Festkörpergelenk 32, der Rahmen 34 und die Lippe 36 umgeben die Öffnung 24 jeweils kreisringförmig, wenn die Öffnung 24 kreisförmig ist.

In der Öffnung 24 ist ein Fensterbauteil 40 angeordnet. Das Fensterbauteil 40 verschließt die Öffnung 24. Die Lippe 36 liegt an einer Randfläche 42 des Fensterbauteils 40 an. Durch den Rahmen 34, die Lippe 36 und die Randfläche 42 des Fensterbauteils 40 werden eine erste Nut 52 und eine zweite Nut 54 begrenzt. Die erste Nut 52 und die zweite Nut 54 weisen bei dem dargestellten Beispiel jeweils einen im Wesentlichen rechteckigen Querschnitt auf und sind voneinander durch die Lippe 36 getrennt. Insbesondere sind die erste Nut 52 für Lot und die zweite Nut 54 für Klebstoff vorgesehen.

Wenn das Fensterbauteil 40 kreisförmig ist bzw. die Randfläche 42 einen Ausschnitt eines Kreiszylinder-Mantels bildet, weisen insbesondere das Festkörpergelenk 32 und die Lippe 36 jeweils im Wesentlichen die Gestalt eines ebenen und dünnen Kreisrings und der Rahmen 34 im Wesentlichen die Gestalt eines Ausschnitts eines Kreiszylinder-Mantels auf. In diesem Fall sind auch die Nuten 52, 54 jeweils kreisringförmig.

Die Wandstärken bzw. Materialstärken des Festkörpergelenks 32, des Rahmens 34 und der Lippe 36 sind jeweils deutlich geringer als die Wandstärke des tubusförmigen Abschnitts 22 des Gehäuses 20 in anderen Bereichen. Die Dicken des Festkörpergelenks 32 und der Lippe 36 werden jeweils in Richtung parallel zur Längsachse 14 des Endoskops 10 gemessen, die Dicke des Rahmens 34 wird radial bzw. senkrecht zur Längsachse 14 gemessen.

Insbesondere betragen die Dicken des Festkörpergelenks 32, des Rahmens 34 und der Lippe 36 jeweils höchstens ein Fünftel der mittleren Wandstärke des tubusförmigen Abschnitts 22 des Gehäuses 20 oder ein Fünftel des an das Festkörpergelenk 32 sich ringförmig anschließenden Bereichs des tubusförmigen Abschnitts 22 des Gehäuses 20. Beispielsweise betragen die Dicken des Festkörpergelenks 32, des Rahmens 34 und der Lippe 36 jeweils höchstens 0,5 mm oder höchstens 0,3 mm oder höchstens 0,2 mm. Abweichend von der Darstellung in Figur 2 können die Dicken des Festkörpergelenks 32, des Rahmens 34 und der Lippe 36 voneinander abweichen.

Abweichend von der Darstellung in Figur 2 kann das Fensterbauteil 40 anstelle zweier ebener und planparalleler Grenzflächen an der Innenseite und an der Außenseite des Gehäuses eine oder zwei konvex und/oder konkav gewölbte Oberflächen aufweisen. In diesem Fall kann das Fensterbauteil 40 die Funktion einer Zerstreuungs- oder Sammellinse aufweisen.

Insbesondere aufgrund unterschiedlicher thermischer Ausdehnungskoeffizienten der Materialien des tubusförmigen Abschnitts 22 des Gehäuses 20 einerseits und des Fensterbauteils 40 andererseits können beim Abkühlen nach dem unten beschriebenen Einlöten des Fensterbauteils 40 in die Öffnung 24 oder beim Autoklavieren des Endoskops 10 mechanische Spannungen zwischen dem tubusförmigen Abschnitt 22 des Gehäuses 20 einerseits und dem Fensterbauteil 40 andererseits auftreten. Diese mechanischen Spannungen können jedoch durch die Elastizität des Festkörpergelenks 32, des Rahmens 34 und der Lippe 36 teilweise abgebaut werden. Dadurch kann die mechanische Belastung des Fensterbauteils 40 reduziert werden. Dies ermöglicht die Verwendung von Glas als Material für das Fensterbauteil 40.

Figur 3 zeigt eine Ausführungsform des proximalen Endes 12 des oben anhand der Figur 1 dargestellten Endoskops 10, die in einigen Merkmalen der oben anhand der Figur 2 dargestellten Ausführungsform ähnelt. Im Unterschied zu der oben anhand der Figur 2 dargestellten Ausführungsform weist die in Figur 3 gezeigte Ausführungsform ein Festkörpergelenk 33 auf, das mit einem S-, Z- oder N-förmigen Querschnitt den Rahmen 34 mit einem radial nach außen angrenzenden massiveren Bereich des tubusförmigen Abschnitts 22 des Gehäuses 20 verbindet. Bei dem dargestellten Beispiel weist das Festkörpergelenk 33 einen Abschnitt auf, der einen Ausschnitt eines Zylindermantels (bei kreisförmiger Öffnung 24: eines Kreiszylinder-Mantels) bildet und koaxial zum Rahmen 34 angeordnet ist. Eine dritte Nut 56 grenzt an den zylindermantelförmigen Abschnitt des Festkörpergelenks 33 an und ist nach außen bzw. zur Umgebung des Gehäuses 20 hin geöffnet.

Im Vergleich zu dem oben anhand der Figur 2 dargestellten Festkörpergelenk 32 kann das in Figur 3 gezeigte Festkörpergelenk 33 bei einem kleineren Bauraum eine größere Elastizität aufweisen. Die dritte Nut 56 kann mit Klebstoff oder einem anderen Material, das elastischer als das Material des Gehäuses 20 ist, aufgefüllt werden, um eine Ansammlung von Schmutz in der dritten Nut 56 zu verhindern.

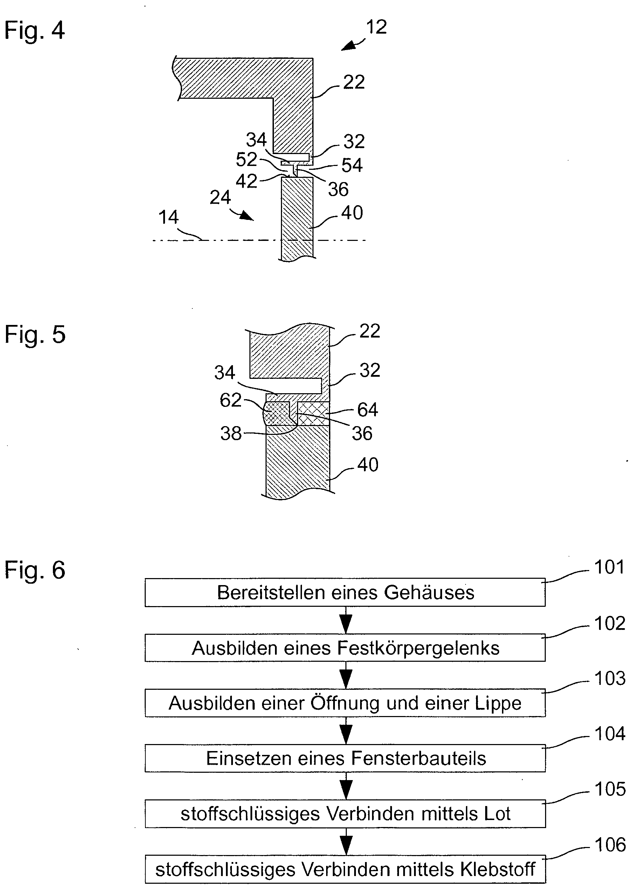

Figur 4 zeigt eine schematische Darstellung einer weiteren Ausführungsform des proximalen Endes 12 des oben anhand der Figur 1 dargestellten Endoskops 10, die in einigen Merkmalen den oben anhand der Figuren 2 und 3 dargestellten Ausführungsformen ähnelt. Die in Figur 4 dargestellte Ausführungsform unterscheidet sich von der oben anhand der Figur 2 dargestellten Ausführungsform vor allem durch eine wesentlich kleinere radiale Ausdehnung des kreisringförmig-ebenen Festkörpergelenks 32. In diesem Fall übernimmt in wesentlich größerem Maß als bei der oben anhand der Figur 2 dargestellten Ausführungsform der außerhalb der Lippe 36 liegende Bereich des Rahmens 34 die Funktion eines Festkörpergelenks.

Jede der oben anhand der Figuren 2 bis 4 dargestellten Ausführungsformen weist eine erste um das Fensterbauteil 40 umlaufende Nut 52 für Lot und eine zweite um das Fensterbauteil 40 umlaufende Nut 54 für Klebstoff auf. In den Figuren 2 bis 4 enthalten die Nuten 52, 54 jedoch noch kein Lot bzw. keinen Klebstoff. Figur 5 zeigt eine schematische Schnittdarstellung eines vergrößerten Ausschnitts der Fügeverbindung zwischen dem tubusförmigen Abschnitt 22 des Gehäuses 20 und dem Fensterbauteil 40 am Beispiel der oben anhand der Figur 4 dargestellten Ausführungsform. Entsprechende Fügeverbindungen sind bei den oben anhand der Figuren 2 und 3 dargestellten Ausführungsformen möglich. In Figur 5 ist ferner erkennbar, dass der radial nach innen gerichtete und an die Randfläche 42 des Fensterbauteils 40 angrenzende Rand 38 der Lippe 36 klingenförmig bzw. mit einem keilförmigen Querschnitt ausgebildet sein kann.

In Figur 5 ist erkennbar, dass Lot 62 in der ersten Nut 52 und Klebstoff 64 in der zweiten Nut 54 jeweils an den Rahmen 34 und an die Randfläche 42 des Fensterbauteils 40 angrenzen. Insbesondere bilden das Lot 62 und der Klebstoff 64 jeweils eine stoffschlüssige Verbindung zwischen dem Rahmen 34 und der Randfläche 42 des Fensterbauteils 40. Die Lippe 36 trennt das Lot 62 und den Klebstoff 64 voneinander. Dadurch ist die Diffusion von Bestandteilen des Lots 62 in den Klebstoff 64 oder umgekehrt stark eingeschränkt. Eine gegenseitige Zerstörung wird dadurch unterbunden oder zumindest verlangsamt.

Sowohl beim Lot 62 als auch beim Klebstoff 64 kann jeweils diejenige Oberfläche, die nicht gleichzeitig Grenzfläche zum Rahmen 34, zur Lippe 36 oder zum Fensterbauteil 40 ist, konvex (wie in Figur 5 für das Lot 62 dargestellt), eben (wie in Figur 5 für den Klebstoff 64 dargestellt) oder konkav ausgebildet sein. Die ebene und fluchtende Ausbildung der Oberfläche des Klebstoffs 64 mit der äußeren Oberfläche des tubusförmigen Abschnitts 22 des Gehäuses 20 und mit der äußeren Oberfläche des Fensterbauteils 40 hat den Vorteil, dass keine Schmutzkanten vorliegen, in denen sich Verunreinigungen ansammeln könnten.

Eine ähnliche Anordnung eines Fensterbauteils, wie sie oben anhand der Figuren 1 bis 5 für das proximale Ende 12 des Endoskops 10 dargestellt ist, ist auch am distalen Ende 11 und/oder am Anschluss 28 für ein Lichtleitkabel möglich.

Figur 6 zeigt ein schematisches Flussdiagramm eines Verfahrens zum Herstellen eines medizinischen Instruments. Obwohl mittels dieses Verfahrens auch medizinische Instrumente herstellbar sind, die sich von den oben anhand der Figuren 1 bis 5 dargestellten Ausführungsformen eines Endoskop unterscheiden, werden nachfolgend beispielhafte Bezugszeichen aus den Figuren 1 bis 5 verwendet, um ein Verständnis des Verfahrens zu erleichtern.

Bei einem ersten Schritt 101 wird ein Gehäuse 20 bereitgestellt. Bei einem zweiten Schritt 102 wird ein Festkörpergelenk 32, 33 an dem Gehäuse ausgebildet. Bei einem dritten Schritt 103 werden eine Öffnung 24 und eine Lippe 36 an dem Gehäuse 20 ausgebildet. Der zweite Schritt 102 und der dritte Schritt 103 können in umgekehrter Reihenfolge oder gleichzeitig ausgeführt werden. Die Öffnung 24 und die Lippe 36 können bei zwei verschiedenen bzw. getrennten Schritten ausgebildet werden.

Bei einem vierten Schritt 104 wird ein Fensterbauteil 40 in die Öffnung 24 im Gehäuse 20 eingesetzt. Bei einem fünften Schritt 105 wird das Fensterbauteil 40 mit dem Gehäuse 20 mittels Lot stoffschlüssig verbunden. Bei einem sechsten Schritt 106 wird das Fensterbauteil 40 mit dem Gehäuse 20 mittels Klebstoff stoffschlüssig verbunden. Der fünfte Schritt 105 und der sechste Schritt 106 können in umgekehrter Reihenfolge oder gleichzeitig ausgeführt werden.

### Bezugszeichen

- 10: Endoskop
- 11: distales Ende des Endoskops 10
- 12: proximales Ende des Endoskops 10
- 14: Längsachse des Endoskops 10

- 20: Gehäuse des Endoskops 10
- 21: Schaft des Endoskops 20
- 22: tubusförmiger Abschnitt des Gehäuses 20
- 24: Öffnung im Gehäuse 20
- 28: Anschluss für Lichtleitkabel
- 32: Festkörpergelenk
- 33: Festkörpergelenk
- 34: Rahmen um Öffnung 24
- 36: Lippe innerhalb des Rahmens 34
- 38: radial innerer Rand der Lippe 36

- 40: Fensterbauteil
- 42: Randfläche
- 52: erste Nut für Lot
- 54: zweite Nut für Klebstoff
- 56: dritte Nut für Klebstoff
- 62: Lot
- 64: Klebstoff

- 101: erster Schritt (Bereitstellen eines Gehäuses)
- 102: zweiter Schritt (Ausbilden eines Festkörpergelenks)
- 103: dritter Schritt (Ausbilden einer Öffnung und einer Lippe)
- 104: vierter Schritt (Einsetzen eines Fensterbauteils)
- 105: fünfter Schritt (stoffschlüssiges Verbinden mittels Lot)
- 106: sechster Schritt (stoffschlüssiges Verbinden mittels Klebstoff)

## Patentansprüche

1. **Medizinisches Instrument** (10) mit:
einem **Gehäuse** (20) mit einer **Öffnung** (24);
einem **Fensterbauteil** (40), das ein transparentes Material aufweist und die Öffnung (24) verschließt;
einem **dünnwandigen Bereich** (32; 33; 34; 36) des Gehäuses (20), der die Öffnung (24) umgibt,
**dadurch gekennzeichnet dass**
der dünnwandige Bereich des Gehäuses (20) ein **Festkörpergelenk** (32; 33) umfasst, das das Fensterbauteil (40) umschließt.

2. Medizinisches Instrument (10) nach Anspruch 1, bei dem
der dünnwandige Bereich eine **Lippe** (36) mit einem Rand (38), der an einer Randfläche (42) des Fensterbauteils (40) anliegt, umfasst.

3. Medizinisches Instrument (10) nach dem vorangehenden Anspruch, bei dem **innerhalb** der Lippe (36) ein **Lot** (62) einen Rahmen (34) um die Öffnung (24) und die Randfläche (42) des Fensterbauteils (40) stoffschlüssig verbindet.

4. Medizinisches Instrument (10) nach einem der Ansprüche 2 und 3, bei dem **außerhalb** der Lippe (36) ein **Klebstoff** (64) einen Rahmen (34) um die Öffnung (24) und die Randfläche (42) des Fensterbauteils (40) stoffschlüssig verbindet.

5. Medizinisches Instrument (10) nach dem vorangehenden Anspruch, bei dem **die minimale Wandstärke** des Gehäuses (20) im Bereich des **Festkörpergelenks** (32; 33) vorliegt.

6. Medizinisches Instrument (10) nach einem der vorangehenden Ansprüche, bei dem das **Fensterbauteil** (40) ein **amorphes Material** aufweist.

7. Medizinisches Instrument (10) nach einem der vorangehenden Ansprüche, wobei
das optische Instrument ein **Endoskop** (10) ist,
das Gehäuse (20) einen **tubusförmigen Abschnitt** (22) aufweist, an dessen Ende die Öffnung (24) angeordnet ist.

8. **Verfahren zum Herstellen** eines medizinischen Instruments (10), bei dem folgende Schritte ausgeführt werden:
Bereitstellen (101) eines **Gehäuses** (20);
Ausbilden (102) einer **Öffnung** (24) und eines **dünnwandigen Bereichs** (32; 33; 34; 36), der die Öffnung (24) umgibt,
wobei der dünnwandige Bereich des Gehäuses (20) ein **Festkörpergelenk** (32; 33) umfasst;
Einsetzen (103) eines **Fensterbauteils** (40), das ein transparentes Material aufweist, in die Öffnung (24),
wobei das Festkörpergelenk (32, 33) das Fensterbauteil (40) umschließt.

9. Verfahren nach dem vorangehenden Anspruch, bei dem der dünnwandige Bereich so ausgebildet wird, dass er eine **Lippe** (36) umfasst, wobei das Fensterbauteil (40) derart in die Öffnung eingesetzt wird, dass ein Rand (38) der **Lippe** (36) **an** einer **Randfläche** (42) des Fensterbauteils (40) anliegt.

10. Verfahren nach dem vorangehenden Anspruch, ferner mit zumindest einem der folgenden Schritte:
stoffschlüssiges Verbinden (104) der Randfläche (42) des Fensterbauteils (40) mit dem Gehäuse (20) mittels eines **Lots** (62), wobei das Lot (62) von innen an die Lippe (36) angrenzt;
stoffschlüssiges Verbinden (105) der Randfläche (42) des Fensterbauteils (40) mit dem Gehäuse (20) mittels eines **Klebstoffs** (105), wobei der Klebstoff (105) von außen an die Lippe (36) angrenzt.

## Claims

1. Medical instrument (10) having:
a housing (20) with an opening (24);
a window component (40), which has a transparent material and closes the opening (24);
a thin-walled region (32; 33; 34; 36) of the housing (20), which thin-walled region surrounds the opening (24),
**characterized in that**
the thin-walled region of the housing (20) comprises a flexure bearing (32; 33) enclosing the window member (40).

2. Medical instrument (10) according to claim 1, wherein
the thin-walled region comprises a lip (36) with an edge (38) bearing on an edge surface (42) of the window component (40).

3. Medical instrument (10) according to the preceding claim, wherein within the lip (36), a solder (62) cohesively connects a frame (34) around the opening (24) and the edge surface (42) of the window component (40).

4. Medical instrument (10) according to either of claims 2 and 3, wherein outside the lip (36), a glue (64) cohesively connects a frame (34) around the opening (24) and the edge surface (42) of the window component (40).

5. Medical instrument (10) according to the preceding claim, wherein the minimal wall thickness of the housing (20) is present in the region of the flexure bearing (32; 33).

6. Medical instrument (10) according to one of the preceding claims, in which the window component (40) comprises an amorphous material.

7. Medical instrument (10) according to one of the preceding claims, wherein
the optical instrument is an endoscope (10),
the housing (20) has a tubular portion (22), at the end of which the opening (24) is arranged.

8. Method for producing a medical instrument (10), in which method the following steps are carried out:
providing (101) a housing (20);
forming (102) an opening (24) and a thin-walled region (32; 33; 34; 36) surrounding the opening (24),
wherein the thin-walled region of the housing (20) comprises a flexure bearing (32; 33);
inserting (103) a window component (40) comprising a transparent material into the opening (24),
wherein the flexure bearing (32; 33) encloses the window member (40) .

9. Method according to the preceding claim, in which method the thin-walled region is formed such that it comprises a lip (36), wherein the window component (40) is inserted into the opening in such a way that an edge (38) of the lip (36) bears on an edge surface (42) of the window component (40).

10. Method according to the preceding claim, moreover with at least one of the following steps:
cohesively connecting (104) the edge surface (42) of the window component (40) to the housing (20) by means of a solder (62), wherein the solder (62) adjoins the lip (36) from the inside;
cohesively connecting (105) the edge surface (42) of the window component (40) to the housing (20) by means of a glue (105), wherein the glue (105) adjoins the lip (36) from the outside.

## Revendications

1. Instrument médical (10) avec :
un boîtier (20) avec une ouverture (24) ;
un composant de fenêtre (40) qui présente un matériau transparent et qui ferme l'ouverture (24) ;
une région à paroi mince (32 ; 33 ; 34 ; 36) du boîtier (20), qui entoure l'ouverture (24),
**caractérisé en ce que**
la région à paroi mince du boîtier (20) comprend une articulation à corps solide (32 ; 33) entourant l'élément de fenêtre (40).

2. Instrument médical (10) selon la revendication 1, dans lequel:
la région à paroi mince comprend une lèvre (36) avec un bord (38) qui s'applique contre une surface de bord (42) du composant de fenêtre (40).

3. Instrument médical (10) selon la revendication précédente, dans lequel, à l'intérieur de la lèvre (36), une brasure (62) relie par engagement par liaison de matière un cadre (34) autour de l'ouverture (24) et la surface de bord (42) du composant de fenêtre (40).

4. Instrument médical (10) selon l'une quelconque des revendications 2 et 3, dans lequel, à l'extérieur de la lèvre (36), un adhésif (64) relie par engagement par liaison de matière un cadre (34) autour de l'ouverture (24) et la surface de bord (42) du composant de fenêtre (40).

5. Instrument médical (10) selon la revendication précédente, dans lequel l'épaisseur de paroi minimale du boîtier (20) se trouve dans la région de l'articulation à corps solide (32 ; 33).

6. Instrument médical (10) selon l'une quelconque des revendications précédentes, dans lequel le composant de fenêtre (40) présente un matériau amorphe.

7. Instrument médical (10) selon l'une quelconque des revendications précédentes, dans lequel l'instrument optique est un endoscope (10), le boîtier (20) présente une portion en forme de tube (22), à l'extrémité de laquelle est disposée l'ouverture (24).

8. Procédé de fabrication d'un instrument médical (10), dans lequel les étapes suivantes sont effectuées:
fourniture (101) d'un boîtier (20) ;
réalisation (102) d'une ouverture (24) et d'une région à paroi mince (32 ; 33 ; 34 ; 36), qui entoure l'ouverture (24),
la région à paroi mince du boîtier (20) comprenant une articulation à corps solide (32 ; 33) ;
insertion (103) dans l'ouverture (24) d'un composant de fenêtre (40) qui présente un matériau transparent,
dans laquelle le articulation à corps solide (32 ; 33) entoure l'élément de fenêtre (40).

9. Procédé selon la revendication précédente, dans lequel la région à paroi mince est réalisée de telle sorte qu'elle comprenne une lèvre (36), le composant de fenêtre (40) étant inséré dans l'ouverture de telle sorte qu'un bord (38) de la lèvre (36) s'applique contre une surface de bord (42) du composant de fenêtre (40).

10. Procédé selon la revendication précédente, comprenant en outre au moins l'une des étapes suivantes :
liaison par engagement par liaison de matière (104) de la surface de bord (42) du composant de fenêtre (40) avec le boîtier (20) au moyen d'une brasure (62), la brasure (62) étant adjacente à la lèvre (36) par l'intérieur ;
liaison par engagement par liaison de matière (105) de la surface de bord (42) du composant de fenêtre (40) avec le boîtier (20) au moyen d'un adhésif (105), l'adhésif (105) étant adjacent à la lèvre (36) par l'extérieur.
